# EUROPÄISCHE PATENTSCHRIFT

(11) **EP 2 294 393 B1**
(45) Veröffentlichungstag und Bekanntmachung des Hinweises auf die Patenterteilung: **03.07.2013**
(21) Anmeldenummer: 09771989.2
(22) Anmeldetag: 08.06.2009
(51) Int. Cl.: G01N 27/24, B65H 63/06, G01N 33/36, B65H 57/04, D01H 13/22, D04B 35/12

(54) **KAPAZITIV ARBEITENDE SENSOREINHEIT ZUR ÜBERWACHUNG DER QUALITÄT VON FASERMATERIAL UND DAMIT AUSGERÜSTETE MASCHINE ZUR HERSTELLUNG VON MASCHENWARE**
CAPACITIVELY OPERATING SENSOR UNIT FOR MONITORING THE QUALITY OF FIBER MATERIAL AND MACHINE EQUIPPED THEREWITH FOR PRODUCING KNITTED FABRICS
UNITÉ DE DÉTECTION À FONCTIONNEMENT CAPACITIF UTILISÉE POUR SURVEILLER LA QUALITÉ DE MATÉRIAUX FIBREUX ET MACHINE ÉQUIPÉE D'UNE TELLE UNITÉ SERVANT À PRODUIRE DES TISSUS MAILLÉS

(30) Priorität: 02.07.2008 DE 102008031108
(43) Veröffentlichungstag der Anmeldung: 16.03.2011
(73) Patentinhaber: SIPRA Patententwicklungs- und Beteiligungsgesellschaft mbH, 72461 Albstadt (DE)
(72) Erfinder: BAUER, Wolfgang, 70806 Kornwestheim (DE); FLAD, Axel, 72393 Burladingen (DE); ABT-SEITEL, Christine, 72458 Albstadt (DE); SCHWAB, Manuel, 72127 Kusterdingen (DE)
(74) Vertreter: Kohler Schmid Möbus
(86) Internationale Anmeldenummer: PCT/DE2009/000807
(87) Internationale Veröffentlichungsnummer: WO 2010/000219

(56) Entgegenhaltungen:
- DE-A1- 1 498 819
- DE-A1- 19 908 236
- DE-B- 1 215 943
- US-A- 1 984 166

## Beschreibung

Die Erfindung betrifft eine Sensoreinheit der im Oberbegriff des Anspruchs 1 angegebenen Gattung und eine mit einer solchen Sensoreinheit ausgerüstete Maschine zur Herstellung von Maschenware.

In der Textilindustrie und hier insbesondere in der Spinnereitechnik ist es allgemein üblich, das zu verarbeitende Fasermaterial laufend auf seine Qualität zu überwachen. Unter "Fasermaterial" werden im Rahmen der vorliegenden Erfindung im Wesentlichen lineare, lang gestreckte, textile Materialien, insbesondere Faserbänder, Garne, Lunten, Flyerlunten, Filamente usw. verstanden, die einem bestimmten Verarbeitungsprozess unterworfen und zu diesem Zweck einem Streckwerk, einer Spinnvorrichtung od. dgl. zugeführt werden sollen.

Die Qualitätsüberwachung derartiger Fasermaterialien kann z. B. mit mechanischen, optischen oder kapazitiven Mitteln erfolgen. Für eine mechanische Überwachung werden Sensoreinheiten verwendet, die eine Rolle mit einer zur Aufnahme des Fasermaterials bestimmten Umfangsnut aufweisen, in die z. B. eine Tastrolle (DE 28 50 775 A1) oder ein Tastfinger (DE 199 50 901 A1) eingreift. Schwankungen in der Dichte oder Masse des Fasermaterials haben entsprechende Schwankungen der Lage der Tastrolle oder des Tastfingers zur Folge und werden mit diesen zugeordneten Wegmessern erfasst. Optische Sensoreinheiten (z. B. DE 32 37 371 A1) weisen aus Lumineszenzdioden und Phototransistoren gebildete Photozellen auf, die vom Fasermaterial durchlaufen werden. Kapazitiv arbeitende Sensoreinheiten schließlich sind mit einem Messkondensator versehen (z. B. EP 0 924 513 A1, DE 199 08 236 A1), der zwei parallel angeordnete Kondensatorplatten aufweist, die einen vom Fasermaterial durchlaufenen Durchgang begrenzen. Als Messgröße dient hier die Kapazität des Messkondensators, die sich entsprechend den Schwankungen im Fasermaterial verändert.

Da die Anwendung von Plattenkondensatoren in der Regel nicht ausreichend genau ist und z. B. durch unvermeidbares Signalrauschen gestört wird, sind auch bereits kapazitiv arbeitende Sensoreinheiten der eingangs bezeichneten Gattung bekannt geworden (z. B. PCT WO 2006/105676 A1), bei denen eine der beiden Kondensatorplatten an ihren Rändern mit zwei isoliert angebrachten Schutzelektroden versehen ist, an die eine sich zeitlich verändernde Spannung angelegt wird.

Sensoreinheiten der beschriebenen Art werden neuerdings auch für so genannte Spinnstrickmaschinen (z. B. PCT WO 2004/079068 A2) und andere maschenbildende Maschinen benötigt, denen anstelle eines üblichen Garns unmittelbar ein in einem Streckwerk oder auf andere Weise verfeinertes Fasermaterial in Form eines Faserbandes, einer Flyerlunte od. dgl. zugeführt wird. Mechanische Sensoreinheiten könnten hier mit Vorteil eingesetzt werden, da sie ein vergleichsweise geringes Signalrauschen verursachen, weitgehend unempfindlich gegen äußere Einflüsse sind und Schwankungen in der Fasermenge (z. B. Zahl der Fasern im Querschnitt eines Fasermaterials) mit ausreichender Genauigkeit abbilden. Ein Nachteil besteht allerdings darin, dass sie aus wenigstens zwei Komponenten, nämlich einem beweglichen Bauteil (Tastrolle, Tastfinger od. dgl.) und einem elektrischen Wegmesser bestehen. Da moderne Strickmaschinen z. B. 96 Stricksysteme aufweisen können und pro Stricksystem wenigstens je eine Sensoreinheit benötigt wird, wären die allein für die Überwachung des Fasermaterials aufzuwendenden Kosten unvertretbar hoch. Optische Sensoreinheiten sind für die Zwecke einer permanenten Überwachung von Fasermaterial an Spinnstrickmaschinen weitgehend ungeeignet, da sie einerseits zu ungenau arbeiten und andererseits empfindlich gegenüber unvermeidbaren Verunreinigungen sind.

Kapazitiv arbeitende Sensoreinheiten der eingangs bezeichneten Gattung haben schließlich zwar den Vorteil, dass sie preisgünstig herstellbar sind. Sie besitzen jedoch den Nachteil, dass die Messsignale eine zu geringe, kaum über ein Grundrauschen hinaus gehende Signalstärke haben, so dass nicht tolerierbare Schwankungen der Faserdichte oder Fasermasse nur ungenau ermittelt werden können. Das gilt zumindest für ihre Anwendung in Maschinen zur Herstellung von Maschenware. Da die Faserdicke und/oder die auf eine Einheitslänge bezogene Fasermasse der auf den Markt angebotenen Fasermaterialien - in deren Längs- bzw. Transportrichtung betrachtet - vergleichsweise häufigen Schwankungen unterworfen sind, müssen bisher entweder Maschenwaren mit ungleichförmiger Qualität, die durch Dick- oder Dünnstellen im Fasermaterial verursacht wird, in Kauf genommen oder aufwendige Maßnahmen getroffen werden, um derartige Schwankungen im Fasermaterial zu vermeiden oder die mit Fehlstellen versehenen Bereiche aus der Maschenware zu entfernen.

In einer Parallelanmeldung derselben Anmelderin vom gleichen Tag ist daher eine nach dem sogenannten 3-Elektroden-Meßprinzip arbeitende Sensoreinheit der eingangs bezeichneten Gattung vorgeschlagen worden. Diese Sensoreinheit enthält eine Meßelektrode, über die das Fasermaterial gleitet, eine der Meßelektrode mit Abstand gegenüberstehende und mit dieser einen Durchgang für das Fasermaterial begrenzende Sendeelektrode und eine der Meßelektrode zugeordnete Schirmelektrode. Dadurch wird der Vorteil erzielt, daß vergleichsweise hohe Signalpegel auch dann erhalten werden, wenn nur sehr kleine kapazitive Änderungen auftreten.

Ausgehend davon besteht das technische Problem der vorliegenden Erfindung darin, die Sensoreinheit der eingangs bezeichneten Gattung so auszubilden, daß das Meßsignal noch weiter verstärkt wird und bei Bedarf an die Bedürfnisse des Einzelfalls angepaßt werden kann. Außerdem soll eine mit einer derartigen Sensoreinheit ausgerüstete Maschine zur Herstellung von Maschenware vorgeschlagen werden.

Zur Lösung dieses Problems dienen die kennzeichnenden Merkmale der Ansprüche 1 und 11.

Die Erfindung bringt den Vorteil mit sich, daß durch den vorstehenden Ansatz eine deutliche Verstärkung des Meßsignals erzielt wird, insbesondere wenn dieser Ansatz eine durch Versuche ermittelbare, optimale relative Lage zu dem über die Meßelektrode gefühten Fasermaterial einnimmt. Auch durch die Form der freien Endfläche des Ansatzes und den Abstand dieser Endfläche vom Fasermaterial läßt sich eine Verstärkung des Meßsignals herbeiführen.

Weitere vorteilhafte Merkmale der Erfindung ergeben sich aus den Unteransprüchen.

Die Erfindung wird nachfolgend in Verbindung mit den beiliegenden Zeichnungen an Ausführungsbeispielen näher erläutert. Es zeigen:
Fig. 1 eine schematische Seitenansicht einer erfindungsgemäßen Sensoreinheit;
Fig. 2 einen Schnitt durch Sensoreinheit längs der Linie II-II der Fig. 1;
Fig. 3 eine perspektivische Darstellung eines praktischen Ausführungsbeispiels der erfindungsgemäßen Sensoreinheit;
Fig. 4 und 5 je einen Vertikalschnitt durch die Sensoreinheit nach Fig. 3 analog zu Fig. 1 und 2;
Fig. 6 eine vergrößerte Einzelheit X der Fig. 4;
Fig. 7 eine Ruridstrickmaschine mit einer erfindungsgemäßen Sensoreinheit; und
Fig. 8 ein weiteres Ausführungsbeispiel der Sensoreinheit in einer der Fig. 5 entsprechenden Ansicht.

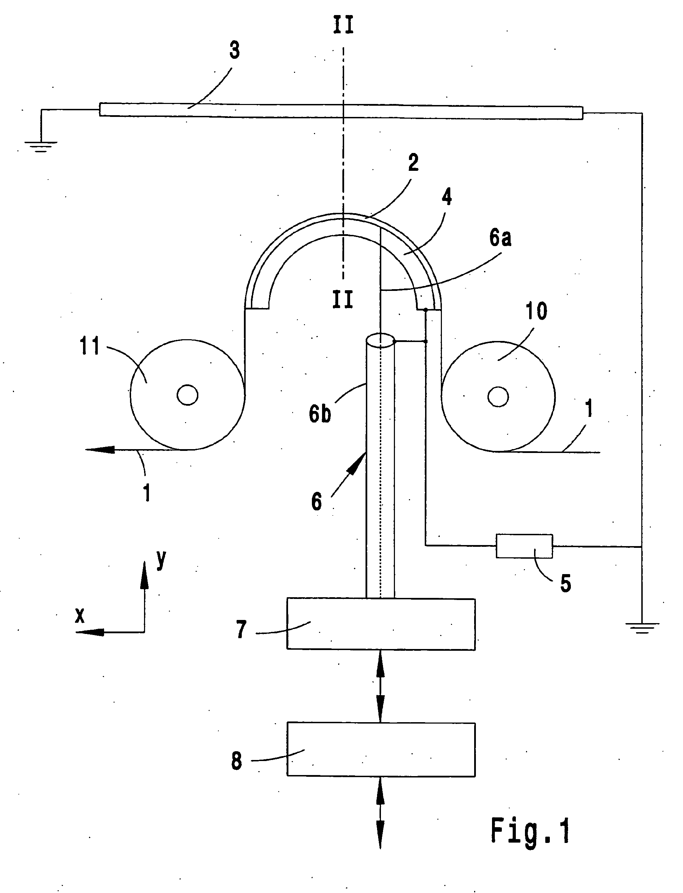

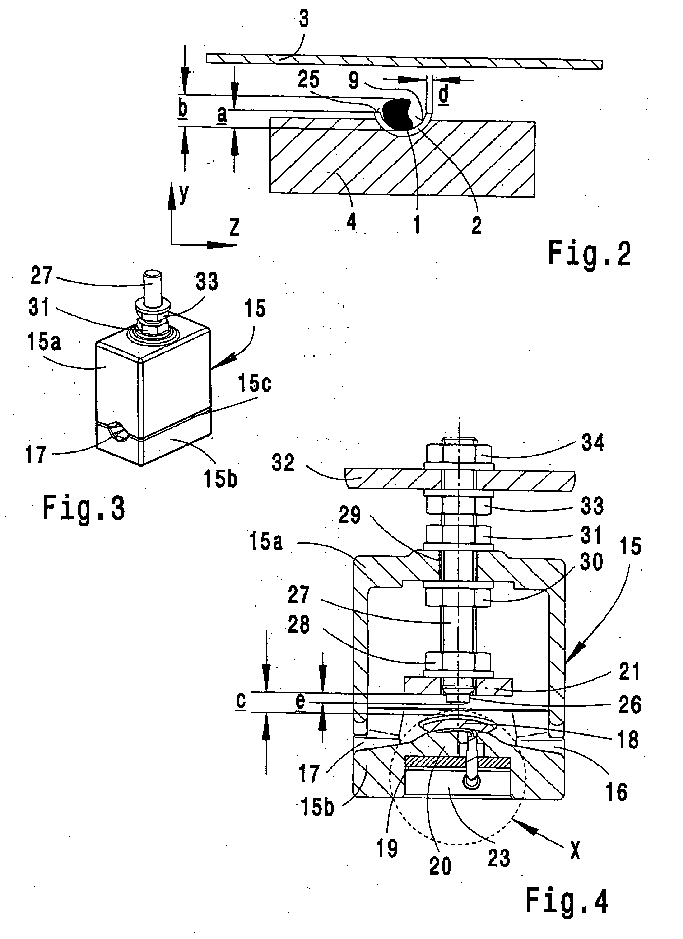

Fig. 1 und 2 zeigen eine erfindungsgemäße, kapazitiv arbeitende Sensoreinheit, die zur Überwachung der Qualität eines als Faserband, Flyerlunte od. dgl. vorliegendes Fasermaterial 1 dient, das in einem kontinuierlichen Prozess z. B. einem nicht dargestellten Streckwerk zugeführt wird. Das Fasermaterial 1 soll z. B. auf seine Homogenität und in diesem Zusammenhang insbesondere auf das Vorhandensein von Dick- oder Dünnstellen überprüft werden, die z. B. durch nicht gleichförmige Fasermassen pro Einheitslänge, eine unterschiedliche Anzahl von Fasern im Querschnitt des Fasermaterials 1 oder auch durch Fremdkörpereinschlüsse im Fasermaterial od. dgl. verursacht sein können. Ungleichmäßigkeiten dieser Art werden nachfolgend allgemein als "Fehlstellen" im Fasermaterial bezeichnet.

Die erfindungsgemäße Sensoreinheit arbeitet im Gegensatz zu bekannten Sensoreinheiten nach dem kapazitiven 3-Elektroden-Messprinzip. Sie enthält zu diesem Zweck eine Elektrodenanordnung, die eine erste, als Messelektrode 2 ausgebildete Elektrode, eine nachfolgend als Sendeelektrode 3 bezeichnete, zweite Elektrode und eine nachfolgend als Schirmelektrode 4 bezeichnete, dritte Elektrode aufweist. Alle drei Elektroden bestehen aus einem elektrisch gut leitenden Material, insbesondere Metall. Wie in Fig. 1 schematisch angedeutet ist, ist die Messelektrode 2 zwischen der Sendeelektrode 3 und der Schirmelektrode 4 angeordnet, die als Kondensatorelektroden aufgefasst werden können. Die Sendeelektrode 3 ist mit dem Signalausgang eines eine Wechselspannung von z. B. ca. 20 kHz liefernden Generators 5 verbunden, dessen anderer Anschluss an die Schirmelektrode 4 angeschlossen ist und auf einem virtuellen Nullpotential liegt, das von der Auswerteelektronik gebildet wird. Zwischen der Messelektrode 2 und der Sendeelektrode 3 befindet sich ein Durchgang für das Fasermaterial 1. Außerdem ist die Messelektrode 2 mit dem Innenleiter 6a eines Koaxialkabels 6 verbunden, dessen Außenleiter 6b mit der Schirmelektrode 4 verbunden ist und daher auf demselben Potential wie diese liegt. Der Innenleiter 6a ist an einen Verstärker 7 angeschlossen, dessen Ausgang mit einer Auswerteelektronik 8 verbunden ist. Die Sendeelektrode 3 wird zusätzlich mit dem als Maschinenmasse bezeichneten Potential verbunden, das z. B. dem Massenpotential einer Rundstrickmaschine, eines Streckwerks od. dgl. entspricht, denen das zu untersuchende Fasermaterial 1 zugeführt wird.

Eine für die Zwecke der Erfindung als besonders wesentlich erachtete Voraussetzung zur Erzielung sicherer Messergebnisse ist eine gleichmäßige Führung des Fasermaterials 1 durch die Sensoreinheit. Erfindungsgemäß ist daher vorgesehen, der Messelektrode 2 die aus Fig. 1 und 2 ersichtliche Form zu geben. Wird der Einfachheit halber angenommen, dass das Fasermaterial 1 in Richtung der X-Achse eines gedachten Koordinatensystems bewegt wird, die Elektroden 2, 3 und 4 entsprechend Fig. 1 in Richtung der Y-Achse des gedachten Koordinatensystems beabstandet sind und dessen Z-Achse in Fig. 1 senkrecht zur Zeichenebene verläuft, dann ist die Messelektrode 2 zunächst so gestaltet, dass sie in der Transportrichtung X einen bogenförmigen, z. B. etwa halbkreisförmigen Verlauf hat und mit ihrer Längsachse parallel zur XY-Ebene angeordnet ist. Außerdem ist die Messelektrode 2 so angeordnet, dass ihr konvex nach außen gewölbtes Teil eine der Sendeelektrode 3 zugewandte Oberseite und ein konkav gekrümmtes Teil eine der Schirmelektrode 4 zugewandte Unterseite der Messelektrode 2 bildet, wie aus Fig. 1 und 2 klar ersichtlich ist. Quer zur bogenförmigen Krümmung, d. h. in der YZ-Ebene gemäß Fig. 2, besitzt die Messelektrode 2 dagegen einen muldenförmigen Verlauf. Eine dadurch gebildete, zur Sendeelektrode 3 hin offene Mulde bildet eine stationäre Gleitfläche 9, längs welcher das Fasermaterial 1 durch die Sensoreinheit gleitet. Vorzugsweise ist die Gleitfläche 9 im Wesentlichen halbzylindrisch ausgebildet, wobei ihr Radius zweckmäßig geringfügig größer als der Durchmesser des Fasermaterials 1 ist. Insgesamt hat die Messelektrode 2 daher das Aussehen einer zur Sendeelektrode 3 hin offenen Dachrinne (Fig. 2), die in ihrer Längsrichtung, d. h. in Transportrichtung X, zusätzlich in der aus Fig. 1 ersichtlichen Weise bogenförmig gekrümmt ist.

Durch die beschriebene Form der Gleitfläche 9 wird eine gute seitliche Führung des Fasermaterials 1 sichergestellt, das in Fig. 2 mit einem kreisförmigen Querschnitt dargestellt ist, aber auch andere Querschnitte besitzen kann.

Damit das Fasermaterial längs der gesamten Messelektrode 2 gleichförmig an der Gleitfläche 9 anliegt, ist an einer Eintrittsseite der Sensoreinheit ein Niederhalter 10 und an der Austrittsseite der Sensoreinheit ein Niederhalter 11 angeordnet. Die Niederhalter 10 und 11 sind bevorzugt als Umlenkelemente ausgebildet, deren Ausgangs- bzw. Eingangsflächen in den Verlängerungen der Messelektrode 2 liegen, damit das Fasermaterial 1 der Messelektrode 2 tangential zugeführt und auch tangential wieder von ihr entfernt wird, wie Fig. 1 zeigt.

Da erfahrungsgemäß die in Transportrichtung X gemessenen Längen üblicher Fehlstellen nur ca. 10 mm bis 20 mm betragen, sollte die X-Richtung gemessene Bogenlänge der Messelektrode 2 einerseits wenigstens etwa genauso groß, andererseits aber auch nicht wesentlich größer sein und höchstens z. B. 40 mm betragen. Um auch kleinste Fehlstellen zu entdecken, wird das Verhältnis Fehlstellenlänge zur Messelektrodenlänge vorzugsweise zwischen 1 : 1 und 1 : 4 gewählt. Außerdem sollte die in Y-Richtung gemessene Tiefe a (Fig. 2) der muldenförmigen Gleitfläche 9 höchstens gleich dem Durchmesser b des Fasermaterials 1, vorzugsweise jedoch höchstens halb so groß wie dieser sein. Dadurch werden ein günstiger Verlauf der elektrischen Feldlinien im Fasermaterial 1 und ein vergleichsweise starkes Messsignal erhalten, ohne dass die seitliche Führung des Fasermaterials 1 verloren geht.

Die Sendeelektrode 3 ist beispielsweise als ebene Metallplatte ausgebildet. Ihre Größe sollte so gewählt sein, dass ihre Parallel-Projektion auf die XZ-Ebene zu einer Fläche führt, die wenigstens genau so groß wie die durch eine entsprechende Parallel-Projektion der Messelektrode 2 erhaltene Fläche ist und diese überdeckt. Diese Maßnahme dient dem Zweck, ein ausreichend ausgedehntes, elektrisches Feld zu erzeugen, das die gesamte Messelektrode 2 erfasst und den gesamten, auf dieser gleitenden Abschnitt des Fasermaterials 1 durchflutet.

Während die Sendeelektrode 3 der konvex gewölbten, nach oben offenen Oberseite der Messelektrode 2 gegenübersteht, ist die Schirmelektrode 4 der konkav gewölbten Unterseite der Messelektrode 2 zugewandt. Die Schirmelektrode 4 ist vorzugsweise so ausgebildet, dass sie die Messelektrode 2 umschließt, ausgenommen an der die Gleitfläche 9 aufweisenden Oberseite. Außerdem muss die Schirmelektrode 4 natürlich elektrisch von der Messelektrode 2 isoliert sein, was durch zwischen beiden angeordnete Isoliermittel sichergestellt wird. Insgesamt hat die Schirmelektrode 4 zweckmäßig eine wirksame, der Unterseite der Messelektrode 2 zugewandte Fläche, die wenigstens genau so groß wie die ihr zugewandte wirksame Fläche der Messelektrode 2 ist. Die Form der Schirmelektrode 4 wird vorzugsweise so gewählt, dass die zwischen ihr und der Sendeelektrode 3 verlaufenden Feldlinien möglichst auch die Messelektrode 2 durchsetzen und nicht seitlich an dieser vorbeilaufen.

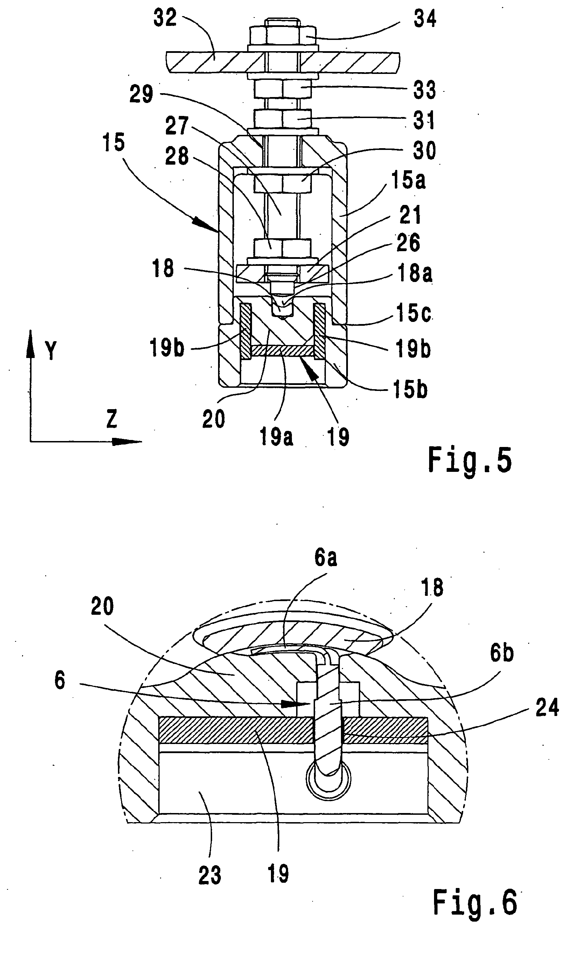

Fig. 3 bis 6 zeigen ein für die praktische Anwendung besonders gut und derzeit für am besten gehaltenes Ausführungsbeispiel der erfindungsgemäßen Sensoreinheit. Diese enthält ein im Wesentlichen geschlossenes Gehäuse 15, das ein Oberteil 15a und ein Unterteil 15b aufweist, die längs einer Trennebene 15c (Fig. 3 und 5) aneinander grenzen, die eine Eintrittsöffnung 16 und eine Austrittsöffnung 17 für das nicht dargestellte Fasermaterial 1 etwa in der Mitte durchsetzt (Fig. 3 und 4). Eine nach Art einer Dachrinne ausgebildete Messelektrode 18, die analog zu Fig. 1 und 2 nach oben offen und in X-Richtung nach oben konvex gewölbt ist, ist im Wesentlichen wie in Fig. 1 und 2 ausgebildet und mit einer muldenförmigen Gleitfläche 18a (Fig. 5) für das Fasermaterial versehen, im Unterschied zu Fig. 1 und 2 aber über einen Bogen von weniger als ca. 180° erstreckt. Die Enden der Messelektrode 18 sind vorzugsweise so angeordnet, dass sie tangential in die Eintritts- bzw. Austrittsöffnung 16 bzw. 17 münden (Fig. 4).

Wie insbesondere Fig. 5 zeigt, sind die Messelektrode 18 und eine ihrer Unterseite zugewandte Schirmelektrode 19 in einen vorzugsweise als Gießharzkörper ausgebildeten Isolator 20 aus einem elektrisch isolierenden Material eingebettet und dadurch einerseits zu einem einstückigen, das Unterteil 15b des Gehäuses 15 bildenden Körper miteinander verbunden, andererseits elektrisch voneinander isoliert. Ferner zeigt Fig. 5, dass die Schirmelektrode 19 U-förmig ausgebildet ist und einen Bodenabschnitt 19a aufweist, an dessen seitlichen Enden zwei in Y-Richtung ragende Seitenwände 19b vorgesehen sind. Die Anordnung ist insbesondere so getroffen, dass die Schirmelektrode 19 die Unterseite und die Seitenteile der Messelektrode 18 U-förmig umgibt und diese daher außer nach oben allseitig umschließt. Dadurch wird eine Konzentration der Feldlinien auf die Messelektrode 18 erreicht und durch Unregelmäßigkeiten der Lunten eine maximale Potentialveränderung an der Messelektrode 18 hervorgerufen.

Die Sensoreinheit nach Fig. 3 bis 6 weist außerdem eine der Oberseite der Messelektrode 18 zugewandte, plattenförmige Sendeelektrode 21 auf, die parallel zur XZ-Ebene angeordnet und an der Innenwand der Decke des topfförmig ausgebildeten Oberteils 15a befestigt ist. Die Sendeelektrode 21 steht der Messelektrode 18 unter Bildung eines Durchgangs für das Fasermaterial mit einem Abstand c (Fig. 4) gegenüber. Dieser Abstand c, vom höchsten Punkt der Messelektrode 18 aus gemessen, sollte einerseits höchstens dem Einbis Zweifachen des Durchmessers des zu überwachenden Fasermaterials 1 betragen, andererseits aber so groß sein, dass auch Verdickungen der Lunten frei passieren können.

Für den Fall, dass die Messelektrode 18 analog zu Fig. 1 und 2 über eine Bogenlänge von ca. 180° erstreckt wird, ist es zweckmäßig, den Niederhaltern 10 und 11 nach Fig. 1 entsprechende Niederhalter auch für die Sensoreinheit nach Fig. 3 bis 6 vorzusehen und diese auf einer gemeinsamen, z. B. durch den Isolator 20 gebildeten oder am Oberteil 15a vorgesehenen Grundplatte zu montieren. Es ist dann möglich, auch die Niederhalter in die vom Gehäuse 15 umschlossene Sensoreinheit zu integrieren.

Schließlich ist es vorteilhaft, das Oberteil 15a und das Unterteil 15b leicht lösbar miteinander zu verbinden, damit ein einfaches Auflegen des Fasermaterials 1 auf die Messelektrode 18 möglich ist. Besonders zweckmäßig ist es, das Oberteil 15a zu diesem Zweck schwenkbar am Unterteil 15b zu befestigen, damit das Gehäuse 15 im Bereich der Trennebene 15c aufgeklappt werden kann.

Im Übrigen ist vorgesehen, die komplett vorgefertigte Sensoreinheit direkt an einer Strickmaschine, einem Streckwerk od. dgl. zu befestigen und dadurch gleichzeitig mit der Maschinenmasse zu verbinden, wie in Fig. 1 schematisch angedeutet ist.

Fig. 4 und 6 zeigen schließlich den Anschluss des auch aus Fig. 1 ersichtlichen Koaxialkabels 6, das z. B. durch eine im Isolator 20 ausgebildete Aussparung 23 hindurch in ein Loch der Schirmelektrode 19 eingesetzt wird. An dieser Stelle kann der Außenleiter 6b durch eine Lötverbindung 24 elektrisch mit der Schirmelektrode 19 verbunden werden. Der Innenleiter 6a wird dagegen z. B. an die Unterseite der Messelektrode 18 angelegt und durch ein leitfähiges Gießharz od. dgl. an dieser fixiert.

Die beschriebene Ausbildung der Sensoreinheit bringt den Vorteil mit sich, dass sie lediglich an einem auf Maschinenmasse liegenden Bauteil befestigt werden braucht und nach dem Anlegen der Generatorspannung zwischen die Schirmelektrode 4, 19 und die Sendeelektrode 3, 21 voll funktionsfähig ist, ohne dass komplizierte Einstellarbeiten erforderlich sind.

Zur Inbetriebnahme der Sensorseinheit nach dem 3-Elektroden-Messprinzip wird mittels des Generators 5 ein Anregungsfeld zwischen der Sendeelektrode 3, 21 (Maschinenmasse) und der Schirmelektrode 4, 19 eingespeist, wobei die Messelektrode 2, 18 zwischen diesen beiden eingeprägten Potentialen angeordnet und über das Fasermaterial 1 an das elektrische Feld angekoppelt ist. Die Messelektrode 2, 18 befindet sich daher auf einem schwimmenden Potential, wobei ihr Abstand zur Schirmelektrode 4, 19 vorzugsweise konstant und kleiner als der Abstand c (Fig. 4) ist. Das über die Messelektrode 2, 18 gleitende Fasermaterial 1 bewirkt daher beim Auftreten von Inhomogenitäten (Fehlstellen) eine Veränderung des elektrischen Feldes und damit auch eine Potentialänderung an der Messelektrode 2 und 18. Durch die beschriebene Gestaltung der Sendeelektrode 3, 21 und der Schirmelektrode 4, 19 und weitere Optimierungen der Sensorgeometrie kann die erzielbare Signalstärke des Messsignals weiter verbessert werden. Dies gelingt insbesondere dann, wenn das vom Generator 5 erzeugte Anregungsfeld (bzw. der Feldlinienverlauf) entsprechend der obigen Beschreibung auf die Messelektrode 2, 18 konzentriert und für eine intensive Durchflutung des Fasermaterials 1 gesorgt wird. Mit anderen Worten sollte der Feldanteil, der nicht das Fasermaterial 1 durchflutet, möglichst klein oder zumindest konstant gehalten werden. In der erfindungsgemäßen Anordnung wird bewirkt, dass im Randbereich der Messelektrode 2, 18 relativ konstante Bedingungen herrschen und Wärmebewegungen, Temperaturdriften der Isolationsmaterialien, insbesondere deren Änderungen der Dielektrizitätskonstante, nicht zu Potentialänderungen der Messelektrode 2, 18 führen. Zu diesem Zweck ist es bei dem anhand der Fig. 1 und 2 beschriebenen Ausführungsbeispiel auch besonders vorteilhaft, eine Wandstärke d der Messelektrode 2, 18 insbesondere in den an die Gleitfläche 9 grenzenden, in Fig. 2 durch das Bezugszeichen 25 gekennzeichneten Bereichen nicht größer als ca. 5 mm zu wählen. Außerdem hat es sich als zweckmäßig erwiesen, den Abstand zwischen der Sendeelektrode 3, 21 und der Messelektrode 2, 18 möglichst klein zu wählen. Dadurch wird eine hohe Feldstärke des Anregungsfeldes bewirkt, die unmittelbar die Messsteilheit positiv beeinflusst.

Der Verstärker 7 und zumindest Teile der Auswerteelektronik 8 für die von der Messelektrode 2, 18 abgegebenen Messsignale können vorzugsweise ebenfalls in der Sensoreinheit bzw. im Gehäuse 15 untergebracht werden. In diesem Fall wird das Messsignal z.B. mit Hilfe von Verstärkern auf einen solchen Signalpegel angehoben, dass es anschließend unter Verzicht auf Koaxialkabel frei von Störbeeinflussungen mit einfachen Kabeln zur Auswerteelektronik 8 weitergeleitet werden kann.

Die Auswertung der Messsignale kann in an sich beliebiger Weise erfolgen, wird bevorzugt aber mit Hilfe von Schaltungsanordnungen durchgeführt, die speziell für das kapazitive 3-Elektroden-Messprinzip entwickelt wurden und zur Detektion extrem kleiner Kapazitätsänderungen geeignet sind. Insbesondere wird in diesem Zusammenhang auf eine Auswertetechnik verwiesen, die aus der Druckschrift DE 100 27 507 C1 bekannt ist, welche zur Vermeidung von Wiederholungen hiermit durch Referenz auf sie zum Gegenstand der vorliegenden Offenbarung gemacht wird.

Erfindungsgemäß ist die Sendeelektrode 21, wie Fig. 4 zeigt, mit einem Ansatz 26 versehen, der in Richtung der Gleitfläche 18a erhaben über ihre z. B. ebene Unterseite vorsteht. Dieser Ansatz 26 liegt mit besonderem Vorteil der höchsten Stelle bzw. dem Scheitelpunkt der konvexen Oberseite der Meßelektrode 18 gegenüber. Außerdem ist der Ansatz 26 an seinem freien Ende vorzugsweise in Richtung der Meßelektrode 18 konvex ausgebildet. Überraschend haben Versuche ergeben, daß durch Wahl der Form des Ansatzes 26 und eines Abstandes e seines freien Endes von der Sendeelektrode 21 der Feldlinienverlauf und damit auch die Größe des erhaltenen Meßsignals optimiert werden kann. Insbesondere eine geeignete Wahl des Verhältnisses der beiden Abstände c und e (Fig. 4) wirkt sich günstig auf den erzielbaren Meßsignalpegel aus.

Damit der Meßsignalpegel bei Bedarf durch Versuche ermittelt und/oder gezielt eingestellt werden kann, ist der Ansatz 26, in Richtung der Gleitfläche 18a betrachtet, vorzugsweise verstellbar relativ zur Sendeelektrode 21 angeordnet. Dies wird nach einem derzeit für am besten gehaltenen und in Fig. 4 dargestellten Ausführungsbeispiel dadurch erreicht, daß der Ansatz 26 durch das eine Ende einer die Sendeelektrode 21 senkrecht durchragenden, vorzugsweise aus einem elektrisch leitenden Material bestehenden Gewindestange 27 gebildet wird, die in eine in der Sendeelektrode 21 vorgesehene Gewindebohrung derart eingedreht ist, daß sie mit ihrem einen Ende in Richtung der Meßelektrode 18 über die Sendeelektrode 21 vorsteht. Außerdem ist die Stirnfläche dieses Endes der Gewindestange 27 konvex ausgebildet. Durch Drehung der Gewindestange 27 in der Gewindebohrung kann somit der Abstand e beliebig eingestellt werden. Zur Fixierung der axialen Lage der Gewindestange 27 in der Gewindebohrung dient eine Mutter 28, die von der im Vergleich zum Ansatz 26 entgegengesetzten Seite der Sendeelektrode 21 her auf die Gewindestange 27 aufgedreht ist.

Mit besonderem Vorteil ist die Gewindestange 27 mit der an ihr befestigten Sendeelektrode 21 in Richtung der Meßelektrode 18 verstellbar im Oberteil 15a des Sensorgehäuses 15 angeordnet. Zu diesem Zweck ist das Oberteil 15a, vorzugsweise in seinem Deckenteil, mit einem Loch 29 versehen, das von einem vom Ansatz 26 entfernten Abschnitt der Gewindestange 27 durchragt wird. Durch axiales Verschieben der Gewindestange 27 in dem Loch 29 können die Abstände der Unterseite der Sendeelektrode 21 und der freien Stirnfläche des Ansatzes 26 von der Meßellektrode 18 gemeinsam eingestellt werden. Die gewünschte Lage der Gewindestange 27 wird dann durch zwei Muttern 30, 31 fixiert, die von entgegengesetzten Seiten des Oberteils 15a her auf die Gewindestange 27 aufgedreht sind.

Schließlich dient die Gewindestange 27 vorzugsweise gleichzeitig zur Befestigung der Sensoreinheit an einer Strickmaschine, einem Strickwerk od. dgl.. Zu diesem Zweck durchragt ein aus dem Sensorgehäuse 15 herausragender Abschnitt der Gewindestange 27 ein Loch in einem geeigneten Maschinenteil 32, das analog zum Oberteil 15a zwischen zwei weiteren auf die Gewindestange 27 augedrehten Muttern 33, 34 fest eingespannt werden kann. Dadurch kann die Sendeelektrode 21 gleichzeitig an die Maschinenmasse gelegt werden.

Bei der Inbetriebnahme der Sensoreinheit dient somit einerseits der in Fig. 4 obere Abschnitt der Gewindestange 27 zu deren Befestigung im Sensorgehäuse 15 sowie als Befestigungsmittel bei der Montage des Sensorgehäuses 15 am Maschinenteil 32. Andererseits kann das in Fig. 4 untere Ende der Gewindestange 27 dazu verwendet werden, die Abstände c und e einzustellen. Dadurch läßt sich eine optimale Funktion der Sensoreinheit herstellen.

Für den Fall, daß die verschiedenen Abstände bekannt sind und nicht mehr verändet werden brauchen, kann der Ansatz 26 natürlich auch starr an der Sendeelektrode 21 angebracht und zusammen mit dieser fest im Sensorgehäuse 15 montiert werden.

Der beschriebene Ansatz 26 ermöglicht nicht nur eine Optimierung des Meßsignals, sondern hebt gleichzeitig auch dessen Pegel derart an, daß es weniger verstärkt werden muss und daher Störungen durch das Grundrauschen reduziert werden.

Die Abmessungen des Ansatzes 26 können unterschiedlich gewählt werden. Als vorteilhaft hat sich erwiesen, wenn der Ansatz 26 einen im wesentlichen der Breite der Meßelektrode 18 entsprechenden Durchmesser hat.

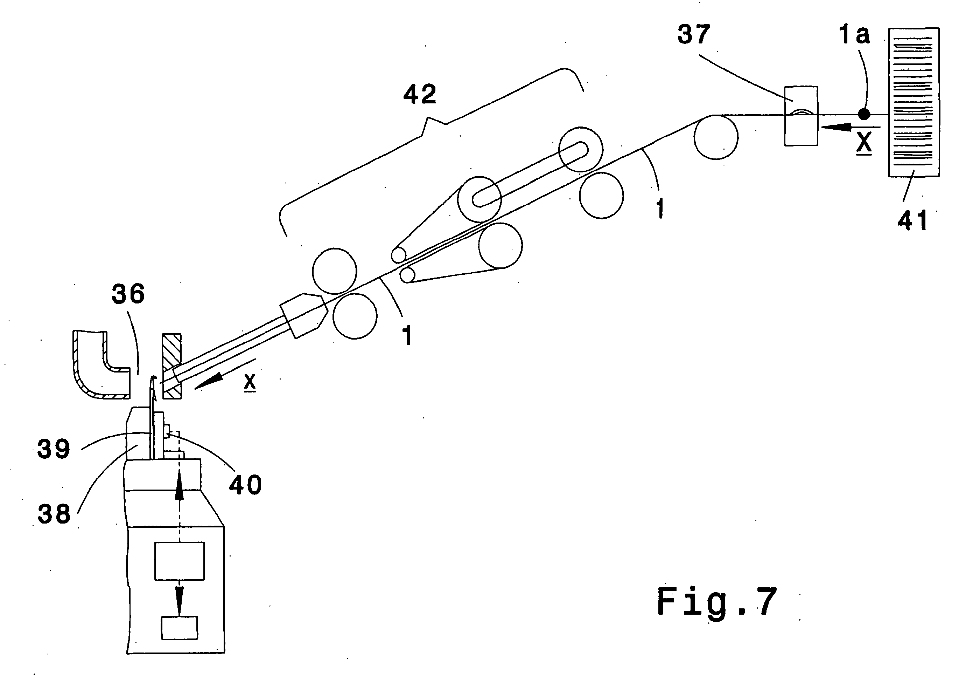

Fig. 7 zeigt eine Teilansicht einer Maschine zur Herstellung von Maschenware am Beispiel einer Rundstrickmaschine mit einer Vielzahl von Maschenbildungsstellen (Stricksystemen) 36, denen wenigstens je eine der beschriebenen, hier mit dem Bezugszeichen 37 bezeichneten Sensoreinheit zugeordnet ist.

Die Rundstrickmaschine enthält einen Nadelzylinder 38, in dem übliche Stricknadeln 39 verschiebbar gelagert sind, die an den Maschenbildungsstellen 36 mit Hilfe von nicht näher dargestellten Schlossteilen 40 in eine zur Aufnahme des Fasermaterials 1 geeignete Faseraufnahmestellung bewegt werden können. Das Fasermaterial 1 wird der Rundstrickmaschine von Vorratsbehältern 41 wie z. B. Kannen, Vorratsspulen od. dgl. aus zugeführt. Zwischen den Vorratsbehältern 41 und den Maschenbildungsstellen 36 sind Streckwerke 42 angeordnet, die in an sich bekannter Weise z. B. drei oder mehr Paare von Streckwalzen aufweisen.

Rundstrickmaschinen der beschriebenen Art sind z. B. aus der Druckschrift PCT WO 2004/079068 bekannt, die hiermit zur Vermeidung von Wiederholungen durch Referenz auf sie zum Gegenstand der vorliegenden Offenbarung gemacht wird.

Ziel der Erfindung ist es, das Auftreten der eingangs erwähnten Fehlstellen in der fertigen Maschenware zu verhindern. Zu diesem Zweck wird das Fasermaterial 1 vor seiner Ankunft an einem Stricksystem 36 mit Hilfe der entsprechend Fig. 1 bis 6 ausgebildeten Sensoreinheit 37 auf die erforderlichen Qualitätsmerkmale, insbesondere Dicke- und Masseschwankungen überprüft, und beim Erkennen einer Fehlstelle, die in Fig. 7 schematisch durch einen Punkt 1a angedeutet ist, wird der Maschenbildungsprozess unterbrochen, die Fehlstelle 1a aus dem Fasermaterial 1 entfernt und der Maschenbildungsprozess dann fortgesetzt.

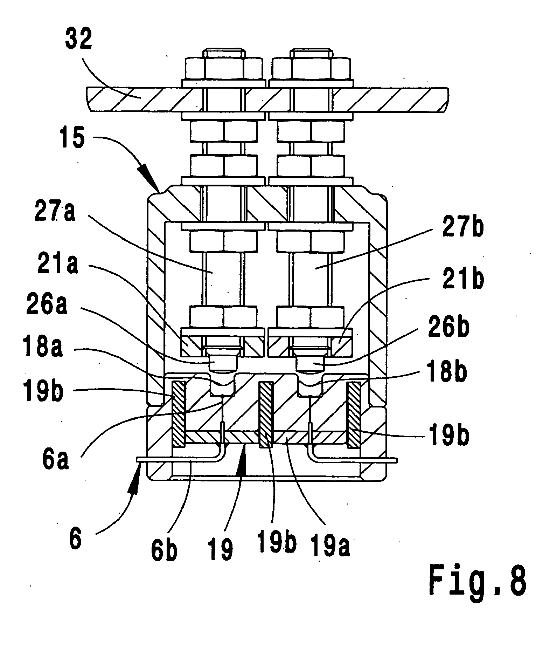

In einem weiteren Ausführungsbeispiel, das in Fig. 8 dargestellt ist, werden in dem zwischen der Sendeelektrode 21 und der Schirmelektrode 19 bestehenden Anregungsfeld mehrere, z. B. entsprechend Fig. 3 bis 6 ausgebildete Meßelektroden 18a, 18b nebeneinander und isoliert voneinander angeordnet. Damit können in einer Sensoreinheit mehrere, parallel zueinander geführte Fasermaterialien 1 gleichzeitig auf Fehlstellen überwacht werden. Die Sendeelektrode 21 und die Schirmelektrode 19 können in der aus Fig. 3 bis 6 ersichtlichen Form mehrfach verwendet und im Gehäuse 15 zu einer Baueinheit zusammengefasst werden, wobei sie lediglich durch den Isolator 20 oder andere geeignete Isoliermittel elektrisch voneinander getrennt werden müssen.

Die Sendeelektrode 21 kann in diesem Ausführungsbeispiel als eine einzige, große Elektrode ausgeführt sein, die mit dem Generator 5 verbunden ist und alle vorhandenen Meßelektroden 18a, 18b überdeckt. Entsprechend kann die Schirmelektrode 19 dadurch ergänzt werden, daß ihr Bodenabschnitt 19a entsprechend verlängert und eine weitere von ihr aufragende Seitenwand 19b vorgesehen wird. Auf diese Weise wird gemäß Fig. 8 eine mehr w- oder kammförmige Schirmelektrode 19 erhalten, wobei die Seitenwände 19b paarweise je eine Meßelektrode 18a, 18b seitlich abschirmen. Besonders vorteilhaft ist es in diesem Ausführungsbeispiel jedoch, jeder Meßelektrode 18a, 18b eine eigene, entsprechend Fig. 3 bis 6 ausgebildete Sendeelektrode 21a, 21b mit einer Gewindestange 27a, 27b zuzuordnen, damit die Abstände c und e (Fig. 3) an jeder Meßelektrode 18a, 18b individuell eingestellt werden können.

Aus der Zusammenfassung der Schirmelektrode 19 gemäß Fig. 8 resultiert außerdem der Vorteil, dass auf dieser direkt die Verstärker 7 (Fig. 1) für die einzelnen Meßelektroden 18a, 18b angebracht werden können, wodurch ein kompakter, integrierter Schaltungsaufbau erhalten wird. Schließlich ist es beim Ausführungsbeispiel nach Fig. 8 in vorteilhafter Weise nur erforderlich, den Außenleiter 6b eines der zu den Meßelektroden 18a, 18b führenden Koaxialkabels 6 an das Schirmpotential anzuschließen und mit der Schirmelektrode 19 z. B. durch Schweißpunkte zu verbinden, da die Außenleiter 6b aller Koaxialkabel 6 in der Auswerteeinheit 8 ohnehin auf ein gemeinsames Potential gelegt werden.

Die Erfindung ist nicht auf die beschriebenen Ausführungsbeispiele beschränkt, die auf vielfache Weise abgewandelt werden können. Das gilt insbesondere für die oben angegebenen Maße für die verschiedenen Krümmungsradien, Abstände und sonstigen Abmessungen, da diese Maße u. a. auch von der Art und den Dimensionen des untersuchten Fasermaterials 1 abhängen. Aus demselben Grund kann es zweckmäßig sein, der Gleitfläche 9 einen mehr kreisförmigen Querschnitt (Fig. 2) oder einen mehr quadratischen oder rechteckigen Querschnitt zugeben. Weiter wäre es möglich, den Sendeelektroden 3, 21 einen an die Krümmung der Messelektroden 2, 18 angepassten bogenförmigen Verlauf zu geben, um dadurch die Feldlinien homogen auf die Messelektroden 2, 18 zu konzentrieren. Außerdem ist klar, dass die Meßelektroden 2, 18 bei der Verwendung von Lunten mit leitfähigen Anteilen, z.B. in Form von Metallfäden, eine dünne Isolationsschicht aufweisen sollten, um elektrische Kurzschlüsse zu vermeiden. Ferner ist klar, dass die Erfindung nicht nur Maschinen zur Herstellung von Maschenware umfasst, die entsprechend Fig. 7 ausgebildet sind, sondern auch z. B. solche, bei denen die Verfeinerung bzw. Verstreckung des Fasermaterials nicht mit Hilfe von Streckwerken, sondern auf andere Weise erfolgt (z. B. PL 350 489 A). Schließlich versteht sich, dass die verschiedenen Merkmale auch in anderen als den beschriebenen und dargestellten Kombinationen angewendet werden können.

## Patentansprüche

1. Kapazitiv arbeitende Sensoreinheit zur Überwachung der Qualität von in einer Transportrichtung bewegtem Fasermaterial (1), enthaltend eine Elektrodenanordnung, die eine Meßelektrode (2, 18), die mit einer als Gleitfläche (9, 18a) für das Fasermaterial (1) ausgebildeten Oberseite und einer Unterseite versehen ist, eine der Gleitfläche (9, 18a) mit Abstand gegenüber stehende und mit dieser einen Durchgang für das Fasermaterial (1) begrenzende Sendeelektrode (3, 21) und eine der Unterseite der Meßelektrode (2, 18) zugeordnete Schirmelektrode (4, 19) aufweist, **dadurch gekennzeichnet, daß** die Sendeelektrode (3, 21) mit einem in Richtung der Gleitfläche (9, 18a) erhaben vorstehenden Ansatz (26) versehen ist.

2. Sensoreinheit nach Anspruch 1, **dadurch gekennzeichnet, daß** der Ansatz (26) in Richtung der Gleitfläche (9, 18a) konvex gewölbt ist.

3. Sensoreinheit nach einem der Ansprüche 1 oder 2, **dadurch gekennzeichnet, daß** die Meßelektrode (2, 18) in Transportrichtung derart bogenförmig gekrümmt ist, daß sie eine konvexe Oberseite und eine konkave Unterseite aufweist, und daß die Gleitfläche (9, 18a) muldenförmig ausgebildet ist.

4. Sensoreinheit nach Anspruch 3, **dadurch gekennzeichnet, daß** der Ansatz (26) einer höchsten Stelle der konvexen Oberseite der Meßelektrode (2, 18) gegenüber steht.

5. Sensoreinheit nach einem der Ansprüche 1 bis 4, **dadurch gekennzeichnet, daß** der Ansatz (26) in Richtung der Gleitfläche (9, 18a) verstellbar angeordnet ist.

6. Sensoreinheit nach einem der Ansprüche 1 bis 5, **dadurch gekennzeichnet, daß** der Ansatz (26) durch eine Stirnfläche am Ende einer Gewindestange (27) gebildet ist, die eine Gewindebohrung der Sendeelektrode (3, 21) durchragt.

7. Sensoreinheit nach einem der Ansprüche 1 bis 6, **dadurch gekennzeichnet, daß** die Meßelektrode (2, 18) und die Schirmelektrode (4, 19) in ein Unterteil (15b) und die Sendeelektrode (3, 21) in ein Oberteil (15a) eines Sensorgehäuses (15) eingebaut sind, das an je einer Eintritts- und Austrittsseite der Meßelektrode (2, 18) je eine Eintritts- und Austrittsöffnung (17, 18) für das Fasermaterial (1) aufweist.

8. Sensoreinheit nach einem der Ansprüche 6 oder 7, **dadurch gekennzeichnet, daß** die Gewindestange (27) verstellbar in einem Loch des Oberteils (15a) des Sensorgehäuses (15) angeordnet ist.

9. Sensoreinheit nach Anspruch 8, **dadurch gekennzeichnet, daß** ein aus dem Oberteil (15a) herausragender Abschnitt der Gewindestange (27) als ein der Montage des Sensorgehäuses (15) an einem Maschinenteil (31) dienendes Befestigungsmittel ausgebildet ist.

10. Sensoreinheit nach einem der Ansprüche 6 bis 9, **dadurch gekennzeichnet, daß** auf die Gewindestange (27) aufgedrehte Muttern (28 bis 34) vorgesehen sind, die der Sicherung der Lage sowohl des erhabenen Vorsprungs (26) relativ zur Sendeelektrode (3, 21) als auch der Gewindestange (27) im Oberteil (15a) des Sensorgehäuses (15) dienen.

11. Maschine zur Herstellung von Maschenware mit wenigstens einer Maschenbildungsstelle (36), Mitteln zur Zuführung von verstrecktem Fasermaterial (1) zur Maschenbildungsstelle (36) und einer Sensoreinheit (37) zur Überwachung der Qualität des Fasermaterials (1), **dadurch gekennzeichnet, daß** die Sensoreinheit (37) nach wenigstens einem der Ansprüche 1 bis 10 ausgebildet ist.

12. Maschine nach Anspruch 11, **dadurch gekennzeichnet, daß** die Sensoreinheit (37) mittels der Gewindestange (27) unmittelbar an einem an Maschinenmasse liegenden Maschinenteil (31) befestigt ist.

## Claims

1. Capacitively operating sensor unit for monitoring the quality of fibre material (1) moved in a transport direction containing an electrode arrangement, which has a measurement electrode (2, 18), which is provided with an upper side configured as slide face (9, 18a) for the fibre material (1) and an underside, a transmission electrode (3, 21) located opposite the slide face (9, 18a) at a distance therefrom and delimiting with this a passage for the fibre material (1) and a shield electrode (4, 19) associated with the underside of the measurement electrode (2, 18), **characterised in that** the transmission electrode (3, 21) is provided with a raised attachment projecting in the direction of the slide face (9, 18a).

2. Sensor unit according to claim 1, **characterised in that** the attachment (26) is convexly curved in the direction of the slide face (9, 18a).

3. Sensor unit according to claim 1 or 2, **characterised in that** the measurement electrode (2, 18) is curved in an arc shape in transport direction in such a manner that it has a convex upper side and a concave underside, and that the slide face (9, 18a) is trough-shaped.

4. Sensor unit according to claim 3, **characterised in that** the attachment (26) is located opposite a highest point of the convex upper side of the measurement electrode (2, 18).

5. Sensor unit according to one of claims 1 to 4, **characterised in that** the attachment (26) is arranged to be adjustable in the direction of the slide face (9, 18a).

6. Sensor unit according to one of claims 1 to 5, **characterised in that** the attachment (26) is formed by a face at the end of a threaded rod (27), which projects through a threaded bore of the transmission electrode (3, 21).

7. Sensor unit according to one of claims 1 to 6, **characterised in that** the measurement electrode (2, 18) and the shield electrode (4, 19) are built into a lower part (15b) and the transmission electrode (3, 21) is built into an upper part (15a) of a sensor housing (15), which respectively has an entry and an exit opening (17, 18) for the fibre material (1) on each entry and exit side of the measurement electrode (2, 18).

8. Sensor unit according to one of claims 6 or 7, **characterised in that** the threaded rod (27) is arranged to be adjustable in a hole of the upper part (15a) of the sensor housing (15).

9. Sensor unit according to claim 8, **characterised in that** a section of the threaded rod (27) projecting out of the upper part (15a) is configured as a fastening element for assembly of the sensor housing (15) on a machine part (31).

10. Sensor unit according to one of claims 6 to 9, **characterised in that** nuts (28 to 34) screwed onto the threaded rod (27) are provided, which serve to secure the position both of the raised projection (26) relative to the transmission electrode (3, 21) and of the threaded rod (27) in the upper part (15a) of the sensor housing (15).

11. Machine for producing knitted goods with at least one stitch-forming location (36), elements for feeding stretched fibre material (1) to the stitch-forming location (36) and a sensor unit (37) for monitoring the quality of the fibre material (1), **characterised in that** the sensor unit (37) is configured according to at least one of claims 1 to 10.

12. Machine according to claim 11, **characterised in that** the sensor unit (37) is fastened directly to a machine part (31) connected to the machine earth.

## Revendications

1. Unité de détection à fonctionnement capacitif destinée à surveiller la qualité d'un matériau fibreux (1) déplacé dans une direction de transport, contenant un arrangement d'électrodes qui comporte une électrode de mesure (2, 18) qui est pourvue d'une face supérieure réalisée sous la forme d'une surface de glissement (9, 18a) pour le matériau fibreux (1) et d'une face inférieure, une électrode émettrice (3, 21) située en face de la surface de glissement (9, 18a) à distance de celle-ci et délimitant avec elle un passage pour le matériau fibreux (1) et une électrode de blindage (4, 19) associée à la face inférieure de l'électrode de mesure (2, 18), **caractérisée en ce que** l'électrode émettrice (3, 21) est pourvue d'un embout (26) en relief qui fait saillie en direction de la surface de glissement (9, 18a).

2. Unité de détection selon la revendication 1, **caractérisée en ce que** l'embout (26) présente une courbure convexe en direction de la surface de glissement (9, 18a).

3. Unité de détection selon une des revendications 1 ou 2, **caractérisée en ce que** l'électrode de mesure (2, 18) est courbée en arc dans la direction de transport de façon à présenter une face supérieure convexe et une face inférieure concave, et que la surface de glissement (9, 18a) est réalisée en forme d'auge.

4. Unité de détection selon la revendication 3, **caractérisée en ce que** l'embout (26) est situé en face du point le plus haut de la face supérieure convexe de l'électrode de mesure (2, 18).

5. Unité de détection selon une des revendications 1 à 4, **caractérisée en ce que** l'embout (26) est disposé de manière déplaçable en direction de la surface de glissement (9, 18a).

6. Unité de détection selon une des revendications 1 à 5, **caractérisée en ce que** l'embout (26) est formé par une face frontale à l'extrémité d'une tige filetée (27) qui traverse un trou fileté de l'électrode émettrice (3, 21).

7. Unité de détection selon une des revendications 1 à 6, **caractérisée en ce que** l'électrode de mesure (2, 18) et l'électrode de blindage (4, 19) sont montées dans une partie inférieure (15b) et l'électrode émettrice (3, 21) dans une partie supérieure (15a) d'un boîtier de capteur (15) qui présente du côté d'entrée, respectivement de sortie, de l'électrode de mesure (2, 18) une ouverture d'entrée, respectivement de sortie (17, 18), pour le matériau fibreux (1).

8. Unité de détection selon une des revendications 6 ou 7, **caractérisée en ce que** la tige filetée (27) est disposée de manière déplaçable dans un trou de la partie supérieure (15a) du boîtier de capteur (15).

9. Unité de détection selon la revendication 8, **caractérisée en ce qu'**une partie de la tige filetée (27) qui dépasse de la partie supérieure (15a) est réalisée sous la forme d'un moyen de fixation servant au montage du boîtier de capteur (15) sur une pièce de machine (31).

10. Unité de détection selon une des revendications 6 à 9, **caractérisée en ce que** des écrous (28 à 34) vissés sur la tige filetée (27), qui servent à fixer la position aussi bien de l'embout en relief (26) par rapport à l'électrode émettrice (3, 21) que de la tige filetée (27) dans la partie supérieure (15a) du boîtier de capteur (15), sont prévus.

11. Machine pour fabriquer du tissu à mailles comportant au moins un poste de formation des mailles (36), des moyens pour amener un matériau fibreux étiré (1) au poste de formation des mailles (36) et une unité de détection (37) pour surveiller la qualité du matériau fibreux (1), **caractérisée en ce que** l'unité de détection (37) est réalisée selon au moins une des revendications 1 à 10.

12. Machine selon la revendication 11, **caractérisée en ce que** l'unité de détection (37) est fixée directement sur une pièce de machine (31) reliée à la masse de la machine au moyen de la tige filetée (27).
